# EUROPEAN PATENT SPECIFICATION

(11) **EP 0 345 694 B1**
(45) Date of publication and mention of the grant of the patent: **15.09.1993**
(21) Application number: 89110139.6
(22) Date of filing: 05.06.1989
(51) Int. Cl.: A61J 9/00

(54) **Nursing bottles**
Saugflaschen
Biberons

(30) Priority: 08.06.1988 BR 8802780; 06.12.1988 BR 6802634 U; 06.12.1988 BR 6802638 U; 06.12.1988 BR 8806409
(43) Date of publication of application: 13.12.1989
(73) Proprietor: Signorini, Alberto, Bairro Sao Conrado Rio de Janeiro, RJ (BR)
(72) Inventor: Signorini, Alberto, Bairro Sao Conrado Rio de Janeiro, RJ (BR)
(74) Representative: Marchitelli, Mauro

(56) References cited:
- EP-A- 0 151 862
- US-A- 2 446 451
- US-A- 2 448 569
- US-A- 3 977 405
- US-A- 3 990 596
- US-A- 4 238 040

## Description

The invention relates to a nursing bottle.

Nursing bottles intended for feeding newborn are well known.

Basically, such "baby bottles" are formed of a body of flask of diverse forms with a threaded neck to which a cap is screwed, thereby fixing the nipple to the neck opening. An overcap or hood is provided to protect the nipple from outside agents. In general, nursing bottles have not had any major developments in their basic form for many years. The traditional bottle shape has been preserved.

The necks of prior art bottles are generally of standard diameters. The neck is formed by narrowing the body in the upper portion so as to form the characteristic shape of a baby bottle. All of the bottles have a thread on their neck portion and, immediately under the same, an expansion to form the body of the flask. This arrangement can encourage the proliferation of germs and bacteria and makes the bottle difficult to clean. Specifically designed brushes are available for cleaning such bottles. Because of the narrowed neck, dishwashers fail to do a proper job.

Another type of nursing bottle is known from US patent No.4 238 040, wherein a threaded adapter ring is disposed within an annular groove of the container, which cooperates with a threaded nipple retainer. The essential features of the present invention which are common to said prior document are set forth in the preamble of Claim 1.

The principal object of the present invention is to solve the above problems creating a new commercial product which constitutes a nursing bottle with the body, or flask, which is completely smooth, has a wide mouth and therefore has the inner portion which is easily accessible for cleaning.

This object is achieved with a nursing bottle having the features indicated in the characterising portion of claim 1. The body or flask is made of a classic plastic glass and is wider than the traditional top of a baby bottle. It tapers slightly from the upper open end to the bottom end. In this respect, the body is slightly conical.

Therefore the body does not have the shape of a bottle but of a glass or cup. The bottle body does not have internal or external threads or grooves. It is formed with smooth walls and without an abrupt increase in diameter. This assures a more hygienic container that requires no special system to clean it. It may be washed with the same ease as a traditional glass. According to the present invention, the nipple-holding cap is not screwed directly on a body or neck thread. Securement is made by a separate counter-cap in a similar manner as in US-4 238 040, but which is however slid upward on the body, from the bottom in the direction of the body top-edge, and caught at the upper edge of the body by a short tooth in low or high relief, yet away from the edge where the nipples rests. This tooth has the characteristic of supporting the screw reaction of the counter-cap and of permitting the cap-and-nipple set to be screwed on tightly. The design of the tooth assures no slippage when the cap is screwed into place, but permits removal of the counter-cap with a slight, downward touch.

When the counter-cap is withdrawn, both cup and counter-cap can be easily washed and sterilized, also allowing the nursing bottle body to be transformed in a classic, smooth-edged glass which the child may thereafter use to learn how to drink out of a normal drinking glass.

The sealing of the nipple against the body is obtained through the pressure between the cap and the counter-cap, this latter been introduced from the bottom to the top, on the frustum body, and embedding on a small tooth, in high or low relief on the outer surface of the body. This tooth retains the counter cap so as to support the action of screwing on the nipple-holding cap which assures a good seal.

It is another object of the invention to provide an accessory that transforms the nursing bottle into a functional breast pump for a mother's bosom.

This object is achieved by using the bottle as a cylinder inside of which runs an especially shaped piston. The piston is held against the mother's breast with a slight pression to form a seal. When the bottle body is pulled away from the breast , a sufficient vacuum is obtained to extract milk from the breast.

After use of the accessory, the piston is removed from the set, the counter-cap is slide on the body, the nipple-holding cap is screwed into place and the baby may be given the mother's milk still warm and without hazard of contamination since the milk has not been transferred to another nursing bottle.

Another object of the invention is to provide a nursing bottle with a spout which, when substituting the traditional nipple to advantage, fills the need of the teething infant to bite on something rigid to help pierce its gums.

Another object of the present invention is to provide the nursing bottle with a double handle which is practical and functional, and which aims to eliminate the drawbacks of cylindrical and smooth nursing bottle bodies used in prior art design of nursing bottles.

A further object of the present invention is to provide a nursing bottle with a system of thermal insulation of the foods contained within the bottle.

These and other objects of the invention would become more apparent to those skilled in the art by reference to the following detailed description when viewed in the light of the accompany drawings in which:
- fig. 1 is a side view of a nursing bottle of the present invention, partly in section,
- fig. 2 is an elevetional view of a member of the bottle of fig. 1,
- fig. 3 is a particular of fig. 2,
- fig. 4 is a particular of fig. 1,
- fig. 5 is an elevetional view of a member of fig. 1,
- fig. 6 is a particular of fig. 5,
- fig. 7 is an elevetional view of a nursing bottle according to the invention in the configuration of a breast pump,
- fig. 8 is a particular of fig. 7,
- fig. 9 is an elevetional view of the nursing bottle according to another embodiment, partly in section,
- fig. 10 is a view taken from arrow X of fig. 9,
- fig. 11 is a plane view of fig. 9,
- fig. 12 is an elevetional view of the nursing bottle according to another embodiment,
- fig. 13 is a particular of fig. 12,
- fig. 14 is a plane view of the particular of fig. 13,
- fig. 15 is an elevetional view of a nursing bottle according to a further embodiment, and
- fig 16 is an elevetional view of a member of the embodiment of fig. 15.

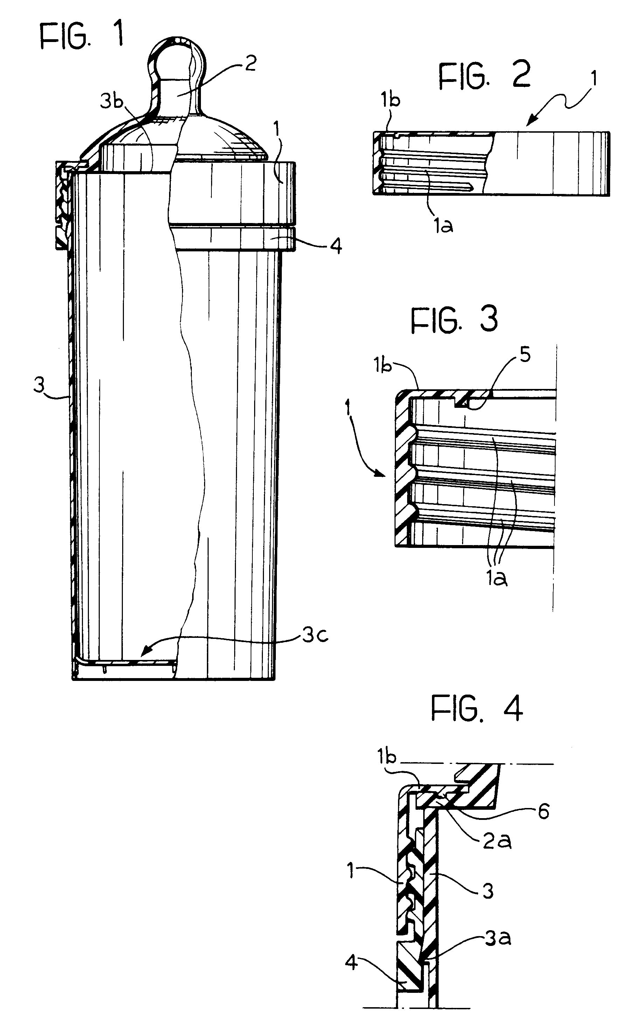

In the following description, identical numerals indicate similar parts in all of the figures. Fig. 1 is a partial section of a nursing bottle and shows the components assembled. Fig. 2 and 3 show a nipple-holding cap 1, having an internal thread 1a and an inwardly directed flange 1b with a downwardly extending circular tooth 5 corresponding to a circular reception groove 6 formed in correspondence on an upper surface of a flange 2a of a nipple 2 made of latex. The flange 1b defines a central opening which receives the central portion nipple 2.

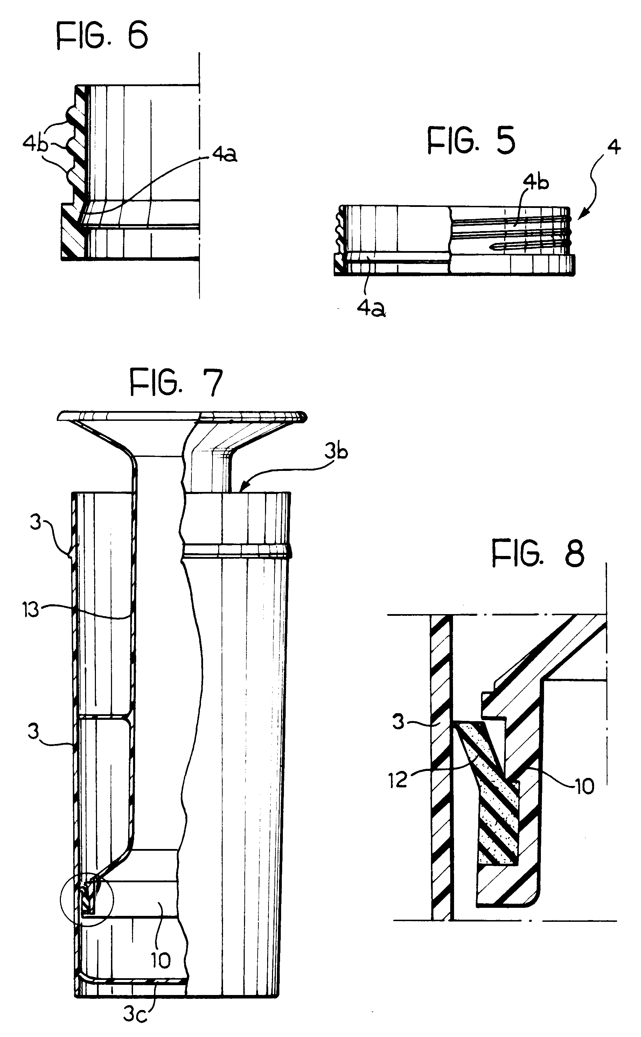

Fig. 5 and 6 show a counter-cap 4 with an internal annular recess 4a apt to receive a ridge 3a of the external wall of a glass 3 made of transparent plastic material. The counter-cap 4 has an external thread 4b corresponding to the internal thread 1a of the nipple-holding cap 1. Fig. 4 shows in detail the assembled configuration of the cap 1, the nipple 2, the glass 3 and the counter-cap 4.

The above configuration assures that nipple 2 is fixed on the cap 1 so that the nipple 2 can be manipulated or screwed on the nursing bottle body in the form of the glass 3 without manual contact, assuring sterility. The sealing of the nipple 2 against the glass 3, which presents a conical form with a mouth 3b larger than the bottom 3c, is performed by the screw action of the counter-cap 1 and by the respective embedding of the tooth 5.

The nursing nipple 2 has a diameter such as to approximate the nimbus of the mother's breast.

It is clear that, in order to assemble the nursing bottle according to the invention, the counter-cap 4 can be slipped over the bottom 3c of the glass 3 and pushed upwardly until it snap engages the ridge 3a of the glass 3 adjacent the rim of the glass, the nipple 2 may be placed against the rim of the glass 3 and the cap 1 may be screwed on the counter-cap 4 in order to press the flange 2a of the nipple 2 against the rim of the glass.

Fig. 7 shows a second embodiment of the nursing bottle in which it is easily transformed into a breast pump. Using the body 3 of the nursing bottle as a cylinder, the above embodiment makes use of a hollow piston 10 having a sealing ring 12 slidable in the glass 3. The piston 13 is integral with an tubular shaft 13 coaxial with the piston 10 and having a cap element 14 in communication with the tubular shaft and with the hollow piston 10 and apt to engage the breast so as to transform the nursing bottle into a breast pump. Placing this breast pump on the nimbus of the mother's bosom so as to cover the mammilla, milk can be extracted from the bosom by holding the edge of the cap element 14 with the thumb and the forefinger of one hand and pulling the bottle body 3 outward with the other hand. After use of the accessory, the piston 10 is removed from the glass 3, the counter-cap 4 is slide on the body, the nipple-holding cap 1 is screwed into place and the baby may be given the mother's milk still warm and without hazard of contamination since the milk has not been transferred to another nursing bottle.

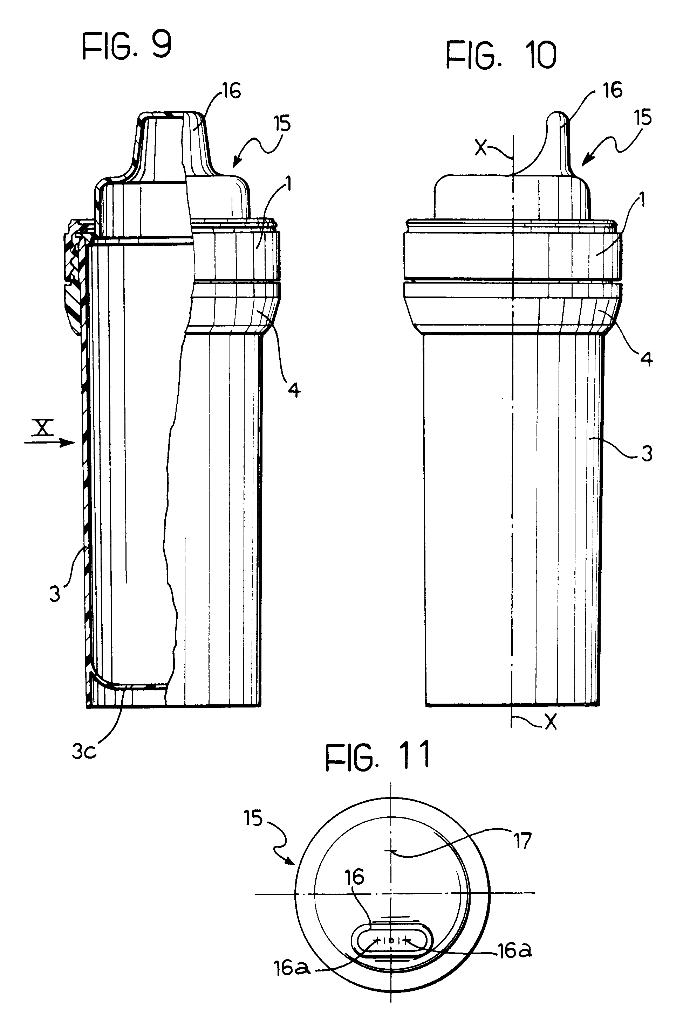

The embodiment shown in fig. 9-11 relates to a rigid nipple 15 which may be used as replacement of the nipple 2 of latex when is necessary for the infant to bite on something rigid when drinking from the nursing bottle. The rigid nipple 15 has a spout 16 eccentrically mounted with respect to axis X-X of the glass 3 and is made of sterilisable and unbreakable atoxic thermoplastic material. As it is clearly shown by fig. 11, the spout 16 presents a plurality of rupturable openings 16a in order to give the mother the ability to increase the number of openings (and therefore the flow of foods) gradually and proportionally to the increasing age of the infant. Moreover, the rigid nipple 15 facilitates the rupturing of the infant's gums during the teething stage.

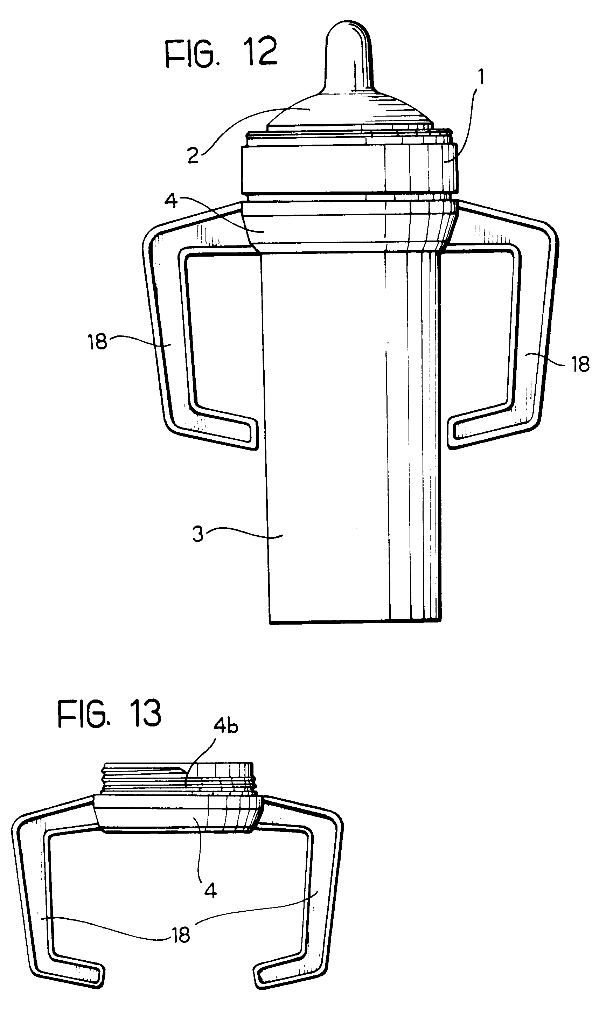

The further embodiment shown in fig. 12-14 relates to a double handled forming part of the counter-cap 4, and allows the infant to hold the bottle naturally, thereby offering the feeling of comfort and safety. The counter-cap 4 presents two handles 18 "C" shaped.

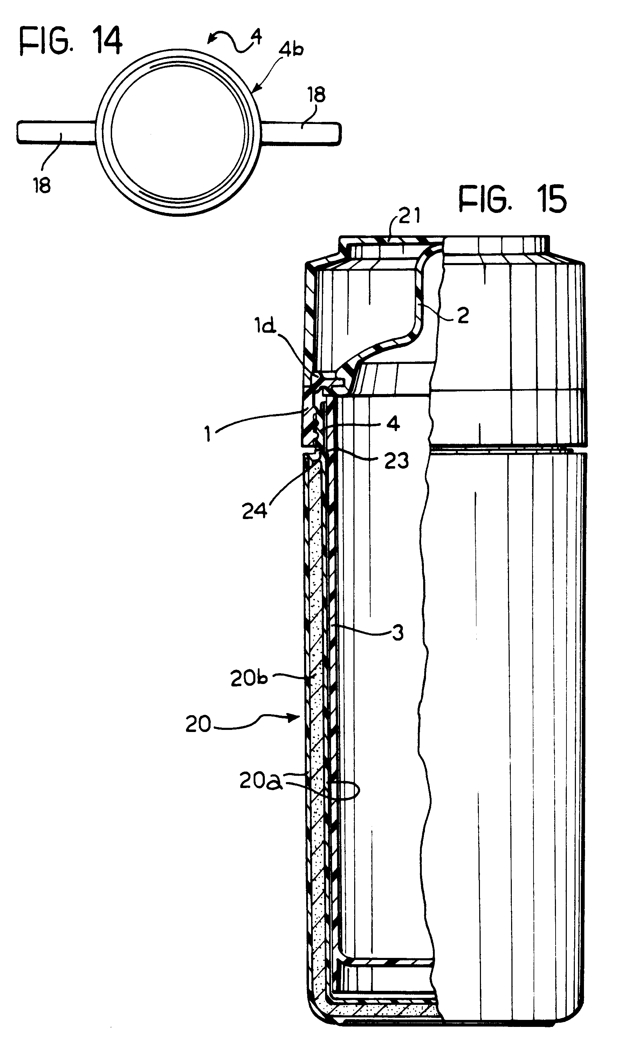

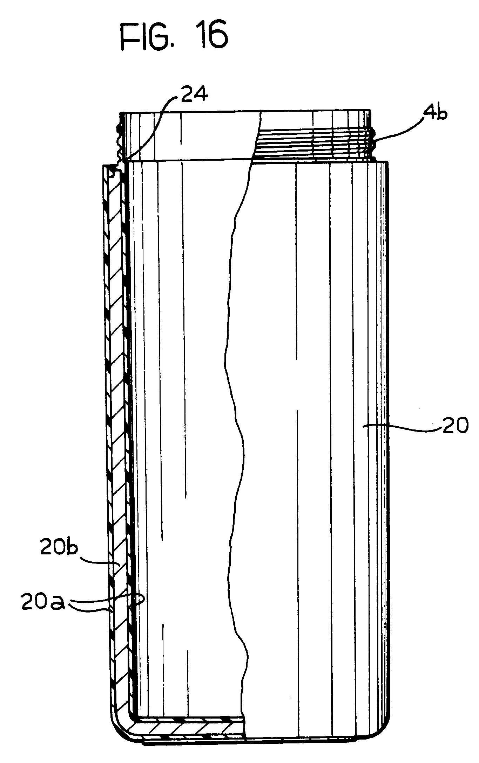

Referring to the last embodiment shown in fig. 15 and 16 the nursing bottle is contained in a thermal holder 20, closed with the nipple-holding cap 1 and covered by an overcap or hood 21.

The thermal holder 20 presents a rim which has a shape similar to that of counter-cap 4 (fig. 5) and is made of boilable thermoplastic material. The overcap 21 is snap-engageble on an anular portion 1d of the cap 1 in order to seal ermetically the inner cup. The thermal holder 20 is made by two cups 20a between which an efficient thermal insulating material 20b is contained.

The thermal accessory presents an outer thread 4b upon which the nipple-holding cap 1 of the nursing bottle can be screwed. The projection 1d upon which the overcap 21 is lodged seals the opening in the nipple 2, thereby preventing the inconvenient leaking experienced with traditional nursing bottles. The large chamber of air created by the overcap 21 around the nipple 2 ensures good thermal isolation at the upper end the nursing bottle.

In the above embodiment, the ridge on the external wall of the glass 3 is substituted with an annular shoulder 23 complementary to an annular shoulder 24 of the counter-cap 4 making part of the thermal holder 20. The cooperation between the annular shoulders 23 and 24 prevents the slippage of thermal holder 20 over the glass 3 in the assembled configuration.

The thermal accessory under consideration allows foods to be kept at the desired temperature for several hours. As it is an integral part of the nursing bottle, it simplifies the daily task of bottle preparation: several can be prepared at once for later feedings, for outings, to assure the mother that other caretakers will be serving the food of her choice.

In a general manner, while some preferred embodiments of the invention as been disclosed, it should be understood that the invention is not limited to such embodiments and there may be changes made in the arrangement, disposition or/and location of the parts without departing from the principal of the present invention as comprehended within the scope of the accompanying claims.

## Claims

1. A nursing device comprising:
- a glass-shaped container (3) having an open end (3b) defined by a rim,
- a first cylindrical collar (4) with a threaded portion (4b) which is adapted to engage the container (3) near its open end (3b),
- a nursing nipple (2) having an external flange (2a) engaging said rim,
- a second collar (1) having a threaded portion (1a) engageable with the threaded portion (4b) of the first collar (4) and having an inwardly directed flange (1b) apt to abut the external flange (2a) of the nursing nipple (2), characterized in that the first cylindrical collar (4) is slidably mounted around the container and in that the container (3) is shaped so that the first collar (4) can be slipped over the bottom (3c) of the container and pushed upwardly until it snugly engages the container (3).

2. Nursing device according to claim 1, wherein the container is conical with a smaller closed bottom.

3. Nursing device according to claim 1, wherein the first cylindrical collar (4) has an internal annular groove (4a) apt to snap engage an annular ridge (3a) on the external wall of the container (3) adjacent to the rim.

4. Nursing device according to claim 2, wherein the nursing nipple (2) has a groove (6) formed in its upper surface and apt to receive an annular tooth (5) extending downwardly from the interiorly directed flange (1b) of the second collar (1).

5. Nursing device according to claim 1, wherein the nursing nipple (2) has a diameter substantially corresponding to the diameter of the rim of the container (3), such diameter being sized to approximate the nimbus of the mother's breast.

6. Nursing device according to claim 1, wherein pumping means are reciprocally mounted in said container (3), said pumping means comprising:
- an hollow piston (10) slidable in the container (3),
- sealing means (12) mounted on the piston (10) to create a seal in the interior of the container (3),
- a tubular shaft (13) coaxial with the hollow piston (10) and in communication with the cylinder defined by the hollow piston (10), the lateral wall and the bottom of the container (3),
- a cup element (14) in communication with the tubular shaft (13) and apt to engage the breast so as to transform the device into a breast pump.

7. Nursing device according to one of claims 1-3, wherein the nursing nipple (15) is rigid and is glass shaped with a spout (16) projecting eccentrically from the bottom.

8. Nursing device according to claim 7, wherein the spout (16) has a plurality of rupturable openings (16a) in order to increase the flow of foods according to the infant's growth.

9. Nursing device according to anyone of claims 1-5, wherein one of the collars (1,4) has a double handle (18) to permit the infant to hold the container (3) without assistance.

10. Nursing device according to anyone of claims 1-5, wherein the first cylindrical collar (4) constitutes a rim of an integral thermal insulating container (20) having an internal shape corresponding to the external shape of the container (3).

## Patentansprüche

1. Saugvorrichtung umfassend:
einen glasförmigen Behälter (3) mit einem durch einen Rand begrenzten, offenen Ende (3b),
einem ersten zylindrischen Kragen (4) mit einem Gewindeabschnitt (4b), der so beschaffen ist, daß er nahe dem offenen Ende (3b) des Behälters (3)liegt,
einem Sauger (2) mit einem äußeren, am genannten Rand anliegenden Flansch (2a),
einem zweiten Kragen (1) mit einem Gewindeabschnitt (1a), der mit dem Gewindeabschnitt (4b) des ersten Kragens (4) in Eingriff bringbar ist und einen nach innen gerichteten Flansch (1b) aufweist, der am äußeren Flansch (2a) des Saugers (2) anliegt, dadurch gekennzeichnet, daß der erste zylindrische Kragen (4) um den Behälter beweglich montiert ist und daß der Behälter (3) so geformt ist, daß der erste Kragen (4) über den unteren Teil (3c) des Behälters geschoben und nach oben bewegt werden kann bis er den Behälter (3) satt untergreift.

2. Saugvorrichtung nach Anspruch 1, dadurch gekennzeichnet, daß der Behälter konisch ist und einen kleineren, geschlossenen Boden aufweist.

3. Saugvorrichtung nach Anspruch 1, dadurch gekennzeichnet, daß der erste zylindrische Kragen (4) eine innere Ringnut (4a) aufweist, in welche eine ringförmige, randnahe Rippe (3a) an der äußeren Wand des Behälters (3) einschnappbar ist.

4. Saugvorrichtung nach Anspruch 2, dadurch gekennzeichnet, daß der Sauger (2) eine Rille (6) aufweist, die in seiner oberen Fläche ausgebildet ist und so beschaffen ist, daß sie einen ringförmigen Zahn (5) aufnimmt, der sich vom nach innen gerichteten Flansch (1b) des zweiten Kragens (1) nach unten erstreckt.

5. Saugvorrichtung nach Anspruch 1, dadurch gekennzeichnet, daß der Sauger (2) einen Durchmesser hat, der im wesentlichen dem Durchmesser des Randes des Behälters (3) entspricht, wobei dieser Durchmesser so dimensioniert ist, daß er jenem des Warzenhofes der mütterlichen Brust gleichkommt.

6. Saugvorrichtung nach Anspruch 1, dadurch gekennzeichnet, daß Pumpmittel im Behälter (3) hin- und herbewegbar montiert sind, wobei diese Pumpmittel umfassen:
einen im Behälter (3) gleitenden Hohlkolben (10), Dichtmittel (12), die am Kolben (10) montiert sind, um im Inneren des Behälters (3) eine Dichtung zu schaffen,
einen rohrförmigen Schaft (13), der zum Hohlkolben (10) koaxial angeordnet ist und mit dem durch den Hohlkolben (10) definierten Zylinder, der Seitenwand und dem Boden des Behälters (3) in Verbindung steht,
ein Schalenelement (14), welches mit dem rohrförmigen Schaft (13) in Verbindung steht und so beschaffen ist, daß es an der Brust angreifen kann, um die Vorrichtung in eine Brustpumpe umzuwandeln.

7. Saugvorrichtung nach einem der Ansprüche 1 bis 3, dadurch gekennzeichnet, daß der Sauger (15) fest und glasförmig ist und einen Schnabel (16) aufweist, der von der Unterseite exzentrisch vorsteht.

8. Saugvorrichtung nach Anspruch 7, dadurch gekennzeichnet, daß der Schnabel (16) meherere durchbrechbare Öffnungen (16a) aufweist, um den Nahrungsfluß entsprechend dem Wachstum des Kindes zu verstärken.

9. Saugvorrichtung nach einem der Ansprüche 1 bis 5, dadurch gekennzeichnet, daß einer der Krägen (1, 4) einen doppelten Handgriff (18) hat, um dem Kind das Halten des Behälters (3) ohne Hilfe zu ermöglichen.

10. Saugvorrichtung nach einem der Ansprüche 1 bis 5, dadurch gekennzeichnet, daß der erste zylindrische Kragen (4) einen Rand eines einstückigen, wärmeisolierenden Behälters (20) bildet, dessen innere Form der äußeren Form des Behälters (3) entspricht.

## Revendications

1. Biberon comportant :
- un réceptacle (3) formé en verre ayant une extrémité ouverte (3b) définie par un rebord ;
- une première collerette cylindrique (4) avec une portion filetée (4b) adaptée pour engager le réceptacle (3) au voisinage de son extrémité ouverte(3b);
- une tétine (2) ayant une bride extérieure (2a) en prise avec ledit rebord ;
- une deuxième collerette (1) comportent une portion filetée (1a) pouvant être engagée sur la portion filetée (4b) de la première collerette (4) et ayant une bride (1b) dirigée vers l'intérieur, apte à venir en butée contre la bride extérieure (2a) de la tétine (2) caractérisée en ce que l'on peut faire glisser la première collerette cylindrique (4) est montée de façon à coulisser autour du réceptacle et en ce que le réceptacle (3) est formé de façon à ce que la première collerette (4) peut être glissée au-dessus du fond (3c) du réceptacle et poussée vers le haut jusqu'à ce qu'elle s'engage en douceur sur le réceptacle (3).

2. Biberon selon la revendication 1, dans lequel le réceptacle est conique avec un fond fermé plus petit.

3. Biberon selon la revendication 1, dans lequel la première collerette cylindrique (4) a une encoche annulaire intérieure (4a) apte à encliqueter un bossage annulaire (3a) sur la paroi extérieure du réceptacle (3) adjacent au rebord.

4. Biberon selon la revendication 2, dans lequel la tétine (2) a une encoche (6) formée sur sa surface supérieure et apte à recevoir une dent annulaire (5) s'étendant vers le bas depuis la bride (1b) dirigée vers l'intérieur de la deuxième collerette (1).

5. Biberon selon le revendication 1, dans lequel la tétine (2) a un diamètre correspondant sensiblement au diamètre du rebord du réceptacle (3), ce diamètre étant dimensionné pour approximer l'aréole du sein de la mère.

6. Biberon selon la revendication 1, dans lequel des moyens de pompage sont montés réciproquement dans ledit réceptacle (3), lesdits moyens de pompage comportant :
- un piston creux (10) pouvant coulisser dans le réceptacle (3) ;
- un moyen d'étanchéité (12) monté sur le piston (10) pour assurer l'étanchéité à l'intérieur du réceptacle (3) ;
- un arbre tubulaire (13) coaxial au piston creux (10) et en communication avec le cylindre défini par le piston creux (10), la paroi latérale et le fond du réceptacle (3) ;
- un élément de couvercle (14) en communication avec l'arbre tubulaire (13) et apte à engager le sein de manière à transformer le dispositif en une pompe à lait.

7. Biberon selon l'une des revendications 1 à 3, dans lequel la tétine (15) est rigide et est formée en verre (16) avec une goulotte projet de façon excentrée depuis le fond.

8. Biberon selon la revendication 7, dans lequel la goulotte (16) a une pluralité d'ouvertures (16a) pouvant être rompues afin d'augmenter le débit de nourriture en fonction de la croissance du nourrisson.

9. Biberon selon l'une quelconque des revendications 1 à 5, dans lequel une des collerettes (1, 4) a une double poignée (18) pour permettre au nourrisson de tenir le réceptacle (3) sans être aidé.

10. Biberon selon l'une quelconque des revendications 1 à 5, dans lequel la première collerette cylindrique (4) constitue un rebord d'un réceptacle (20) à isolement thermique intégré ayant une forme intérieure correspondant à la forme extérieure du réceptacle (3).
